(19) Europäisches Patentamt
European Patent Office
Office européen des brevets

(11) Veröffentlichungsnummer: **0 212 509**
**A1**

(12) # EUROPÄISCHE PATENTANMELDUNG

(21) Anmeldenummer: **86111019.5**

(22) Anmeldetag: **09.08.86**

(51) Int. Cl.4: **C25B 3/02**, C07C 43/32

(30) Priorität: **14.08.85 DE 3529074**

(43) Veröffentlichungstag der Anmeldung: **04.03.87 Patentblatt 87/10**

(84) Benannte Vertragsstaaten: **BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Degner, Dieter, Dr.**
**Kurpfalzstrasse 8**
**D-6701 Dannstadt-Schauernheim(DE)**
Erfinder: **Steckhan, Eberhard, Prof. Dr.**
**Jungholzweg 26**
**D-5309 Meckenheim(DE)**

(54) **Verfahren zur Herstellung von Benzoesäureorthoestern sowie Verbindungen dieser Klasse.**

(57) Herstellung aromatischer Carbonsäureorthoester der Formel I

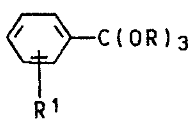

I,

(mit R = $C_1$-$C_4$-Alkyl und $R^1$ = H, Halogen, Alkyl, Aryl, Heteroaryl, Alkoxi, Aryloxi, Acyl, Acyloxi oder CN) durch Elektrooxidation von Benzolderivaten der Formel II

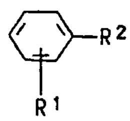

II,

(mit $R^2$ = $CH_3$ oder $CH(OR_2)$)

in Gegenwart eines Alkohols ROH, einer halogenierten Triarylaminverbindung und einer Base sowie neue 4-tert.Butoxibenzoesäure-o-trialkylester mit $C_1$-bis $C_4$-Alkylresten.

4-tert.Butoxibenzoesäure-o-trialkylester sind Riechstoffe und dienen weiterhin zur Herstellung von p-Hydroxibenzoesäureestern.

EP 0 212 509 A1

# Verfahren zur Herstellung von Benzoesäureorthoestern sowie Verbindungen dieser Klasse

Die Erfindung betrifft ein Verfahren zur Herstellung von Benzoesäureorthoestern durch Elektrooxidation von Benzaldehyddialkylacetalen und/oder Toluolderivaten sowie neue 4-tert.Butoxibenzoesäure-o-trialkylester.

Aus J. Chem. Soc. Perkin I, 1978, 708-715 und der DE-PS 28 48 397 ist bekannt, daß man Toluole durch anodische Oxidation in Gegenwart von Methanol selektiv in die entsprechenden Benzaldehyddimethylacetale überführen kann. Eine elektrochemische Oxidation der Benzaldehyddialkylacetale zu den entsprechenden Orthoestern gelingt jedoch auch bei Anwendung eines sehr hohen Stromüberschußes nur mit sehr geringer Selektivität.

Der Erfindung lag die Aufgabe zugrunde, ein Verfahren zur Verfügung zu stellen, das es erlaubt, unsubstituierte oder substituierte Benzoesäure-o-trialkylester mit hoher Selektivität herzustellen.

Demgemäß wurde gefunden, daß man aromatische Carbonsäureorthoester der allgemeinen Formel I

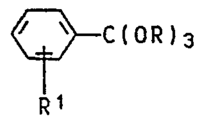

I,

in der R einen Alkylrest mit 1 bis 4 C-Atomen bedeutet und $R^1$ für ein Wasserstoffatom, Halogenatom oder einen Alkyl-, Aryl-, Heteroaryl-, Alkoxi-, Aryloxi-, Acyl-, Acyloxi-oder Cyanrest steht, besonders vorteilhaft herstellt, wenn man Benzolderivate der allgemeinen Formel II

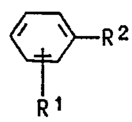

II,

in der $R^2$ für einen Methylrest oder einen Rest der Formel $-CH(OR)_2$ steht, in Gegenwart

a) eines Alkohols der Formel ROH, und

b) Triarylaminverbindung der allgemeinen Formel III

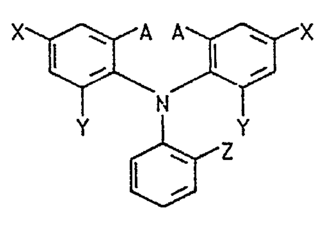

III,

in der beide A entweder Wasserstoffatome oder zusammen eine Einfachbindung, X ein Halogenatom oder einen $H_3COC$-, $NO_2$-oder NC-Rest, Y ein Wasserstoffatom, einen $-NO_2$-oder $CH_3COC$-Rest oder ein Halogenatom und Z ein Wasserstoffatom, einen $-NO_2$-Rest oder ein Halogenatom bedeuten, und

c) einer Base elektrolysiert.

In den Benzaldehyddialkylacetalen und den Toluolderivaten der Formel II steht R für einen Alkylrest mit 1 bis 4 C-Atomen, vorzugsweise für einen Methyl-oder Ethylrest. Als Reste $R^1$ kommen neben Wasserstoffatomen verzweigte oder unverzweigte Alkylreste, z.B. solche mit 1 bis 10, insbesondere 1 bis 6 C-Atomen, in Betracht. Alkoxigruppen vorteilhaft mit 1 bis 6 C-Atomen sind z.B. Methoxi-oder Ethoxigruppen. Aryl-und Aryloxigruppen sind z.B. Phenyl-und Phenoxigruppen. Als Acyl-und Acyloxigruppen seien z.B. $-CO-CH_3$ oder $-COOCH_3$ genannt. Heteroarylreste sind z.B. 5-oder 6-gliedrige Heteroaromaten mit 1 oder 2 Heteroatomen wie Stickstoff, Sauerstoff oder Schwefel. Weiterhin bedeutet $R^1$ Halogenatome, vorzugsweise Chlor oder Brom, oder einen Cyanrest CN. Der Rest $R^1$ steht in meta-, ortho-oder vorzugsweise para-Stellung.

Bevorzugte Ausgangsstoffe der Formel II sind z.B. Benzaldehyddimethylacetal, Benzaldehyddiethylacetal, 4-Methylbenzaldehyddimethylacetal, 4-tert.Butylbenzaldehyddimethylacetal, 4-tert.-Butoxibenzaldehyddimethylacetal, 4-Methoxibenzaldehyddiméthylacetal, 4-Brombenzaldehyddimethylacetal, 4-Chlorbenzaldehyddimethylacetal, p-Xylol, 4-tert.Butyltoluol.

Alkanole der Formel ROH, in der R die obengenannte Bedeutung hat, sind z.B. Ethanol, Propanol, Isopropanol, Butanol, Isobutanol und vorzugsweise Methanol.

Triarylaminverbindungen der Formel III sind Verbindungen folgender Struktur

, 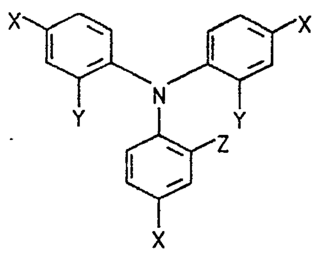 oder 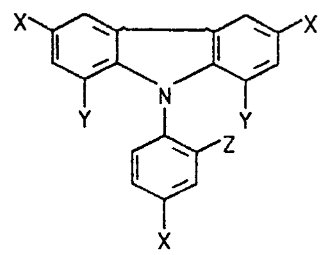 ,

die als Halogenatome z.B. F-, Cl-oder Br-Atome enthalten. Verbindungen der Formel III sind z.B. Tris-(4-bromphenyl)-amin, Bis-(4-bromphenyl)-(2,4-dibromphenyl)-amin, Bis-(2,4-dibromphenyl)-4-bromphenylamin, Tris-(2,4-dibromphenyl)-amin, Tris-(4-chlorphenyl)-amin, Bis-(4-chlorphenyl)-(2,4-dichlorphenyl)-amin, Bis-(2,4-dichlorphenyl)-(4-chlorphenyl)-amin und Tris-(2,4-dichlorphenyl)-amin, von denen Tris-(2,4-dibromphenyl)-amin und Tris-(2,4-dichlorphenyl)-amin bevorzugt sind.

Als Basen kommen Alkali-bzw. Erdalkalicarbonate oder -hydrogencarbonate wie $LiCO_3$, $Na_2CO_3$, $NaHCO_3$, $K_2CO_3$, $KHCO_3$ oder $MgCO_3$ in Betracht. Bevorzugte Basen sind Alkali-bzw. Erdalkalihydroxide wie LiOH und insbesondere NaOH, KOH bzw. $Mg(OH)_2$. Besonders bevorzugt werden Alkoholate der allgemeinen Formel IV

$Me(OR)_x$ IV,

worin Me ein Alkali-oder Erdalkalimetall, X die Zahlen 1 oder 2 und R einen Alkylrest mit 1 bis 4 C-Atomen darstellen, also z.B. $NaOCH_3$, $NaOC_2H_5$ oder $KOCH_3$, als Basen verwendet.

Dem Elektrolyten werden die in der organischen Elektrochemie üblichen Leitsalze, wie Salze der Tetrafluorborsäure, Salze von Alkyl-oder Arylsulfonsäuren oder Salze von Alkylschwefelsäuren sowie Salze der Perchlorsäure zugesetzt. Zur Erhöhung der Löslichkeit des Elektronenüberträgers können dem Elektrolyten Kolösungsmittel zugesetzt werden. Als Kolösungsmittel kommen z.B. Halogenkohlenwasserstoffe, wie Methylenchlorid, Dichlorethan, 1,2-Dichlorpropan oder Nitrile, wie Acetonitril in Betracht. Die Kolösungsmittel werden dem Alkanol z.B. in Mengen bis zu 60 Gew.-Teilen pro 100 Gew.-Teile Alkanol zugegeben.

Das erfindungsgemäße Verfahren benötigt keine besondere Elektrolysezelle, bevorzugt wird eine ungeteilte Durchflußzelle eingesetzt. Als Anoden können alle an sich üblichen Anodenmaterialien verwendet werden, die unter den Elektrolysebedingungen stabil sind, wie Edelmetalle, z.B. Gold oder Platin. Bevorzugt verwendet man Graphit sowie glasartigen Kohlenstoff. Als Kathodenmaterial kommen u.a. Graphit, Eisen, Stahl, Nickel oder auch Edelmetalle, wie Platin, in Betracht.

Der bei der Elektrooxidation eingesetzte Elektrolyt hat beispielsweise folgende Zusammensetzung:

1 bis 70 Gew.% Ausgangsverbindung der Formel II

30 bis 96 Gew.% Alkanol mit oder ohne Kolösungsmittel

0,5 bis 5 Gew.% Triarylaminverbindung der Formel III

0,5 bis 4 Gew.% Leitsalz

0,05 bis 3 Gew.% Base.

Man elektrolysiert bei Stromdichten von 0,25 bis 10 $A/dm^2$, vorteilhaft von 0,25 bis 5 $A/dm^2$, bevorzugt bei 0,5 bis 3 $A/dm^2$. Die Ladungsmenge beträgt 2,5 bis 25, vorzugsweise 3 bis 20 F pro Mol Ausgangsstoff.

Die Elektrolysetemperaturen sind nach oben hin durch den Siedepunkt des Alkanols bzw. des Kolösungsmittels begrenzt. Zweckmäßigerweise elektrolysiert man bei Temperaturen von z.B. 10 bis 5°C unterhalb des Siedepunktes des Elektrolyten. Bei Verwendung von Methanol wird z.B. bei Temperaturen bis 60°C, vorzugsweise bei 20 bis 60°C, elektrolysiert. Es wurde überraschend festgestellt, daß das erfindungsgemäße Verfahren die Möglichkeit bietet, die Ausgangsverbindungen der Formel II sehr weitgehend umzusetzen, ohne daß es zu einer erheblichen Verschlechterung der Selektivität der Elektrooxidation kommt. Das Verfahren kann sowohl diskontinuierlich als auch kontinuierlich durchgeführt werden.

Die Aufarbeitung der Elektrolysenausträge nimmt man nach an sich bekannten Methoden vor. Zweckmäßigerweise wird der Elektrolyseaustrag destillativ aufgearbeitet. Überschüssiges Alkanol und evtl. eingesetztes Kolösungsmittel werden zunächst abdestilliert, Leitsalz und Triarylaminoverbindung werden abfiltriert und die Benzoesäureorthoester werden gereinigt, z.B. durch Destillation. Alkanol, Kolösungsmittel, Leitsalz und Triarylaminoverbindung sowie nicht umgesetzte Verbindungen der Formel II können zur Elektrolyse zurückgeführt werden. Nach 2500 regenerativen Zyklen konnte noch kein nennenswerter Verlust an Triarylaminverbindung festgestellt werden.

Bei der Durchführung des Verfahrens hat es sich gezeigt, daß man die Elektrooxidation über längere Zeit durchführen kann, ohne daß es zu Elektrodenproblemen oder zu einer Verschlechterung der Selektivitäten bei der Elektrooxidation kommt. Das ist bemerkenswert, da der technischen Durchführbarkeit einer organischen Elektrolyse oft das Verhalten der Elektroden, die insbesondere bei gleichzeitiger Rückführung des Elektrolyten zu einer höchst unerwünschten Belagbildung neigen, entgegensteht.

Die nach dem erfindungsgemäßen Verfahren erhältlichen Benzoesäureorthoester dienen als Riechstoffe. 4-tert.Butoxibenzoesäuretrimethyl-und -triethylester haben sehr interessante campfrig und eugenolartige Geruchseigenschaften. Weiterhin werden sie als Ausgangsstoffe für die Herstellung von p-Hydroxibenzoesäureestern, die Lebensmitteln zur Stabilisierung zugesetzt werden, verwendet.

Beispiel 1

Elektrosynthese von 4-tert.Butoxibenzoesäure-o-trimethylester I/1 Zelle : ungeteilte Zelle mit 9 Elektroden

Anode : Graphit

Elektrolyt :

894 g (33,0 Gew.%) 4-tert.Butoxibenzaldehyddimethylacetal II/1

8,9 g (0,33 Gew.) Tris-(2,4-dibromphenyl)-amin

8.9 g (0,33 Gew.%) $KSO_3C_6H_5$

9,2 g (0,34 Gew.%) $NaOCH_3$

1.787,2 g (66 Gew.%) $CH_3OH$

Kathode : Graphit

Stromdichte : 3,3 $A/dm^2$

Elektrolyse mit 3,4 F/Mol 4-tert.Butoxibenzaldehyddimethylacetal II/1

Temperatur : 22 bis 24°C

Durchfluß durch Zelle: 200 l/h

Aufarbeitung: Nach Beendigung der Elektrolyse wurde $CH_3OH$ abdestilliert, das ausgefallene Salz abfiltriert und der Rückstand bei 4 mbar Kopfdruck fraktioniert destilliert. Die Hauptfraktion destillierte bei 140°C (Kopftemperatur) über. Man erhielt 137,3 g 4-tert.Butoxibenzaldehyddimethylacetal II/1, 473,9 g 4-tert.Butoxibenzoesäure-o-trimethylester I/1 und 44,6 g 4-tert.Butoxibenzoesäuremethylester.

Ergebnis:

Umsatz 4-tert.Butoxibenzaldehyddimethylacetal II/1: 85 %

Ausbeute 4-tert.Butoxibenzoesäure-o-trimethylester I/1: 47%

Selektivität 4-tert.Butoxibenzoesäure-o-trimethylester I/1: 55%

Beispiel 2

Elektrosynthese von 4-Methoxibenzoesäure-o-trimethylester I/2 Zelle : ungeteilte Zelle mit 11 Elektroden

Anode : Graphit

Elektrolyt :

869 g (32,2 Gew.%) 4-Methoxibenzaldehyddimethylacetal II/2

9,2 g (0,34 Gew.) Tris-(2,4-dibromphenyl)-amin

9,2 g (0,34 Gew.%) $KSO_3C_6H_5$

9,2 g (0,34 Gew.%) $NaOCH_3$

1.782 g (66,78 Gew.%) $CH_3OH$

Kathode : Graphit

Stromdichte : 2 $A/dm^2$

Elektrolyse mit 6 F/Mol 4-Methoxibenzaldehyddimethylacetal II/2

Temperatur : 33 bis 36°C

Durchfluß durch Zelle: 200 l/h

Aufarbeitung: analog Beispiel 1. Die fraktionierte Destillation wurde bei 2 mbar Kopfdruck und Kopftemperaturen von 90 bis 150°C durchgeführt. Hierbei erhielt man 67 g 4-Methoxibenzaldehyddimethylacetal II/2, 492 g 4-Methoxibenzoesäure-o-trimethylester I/2 und 135 g 4-Methoxibenzoesäuremethylester

Ergebnis:

Umsatz 4-Methoxibenzaldehyddimethylacetal II/2: 92 %

Ausbeute 4-Methoxibenzoesäure-o-trimethylester I/2: 49 %

Selektivität 4-Methoxibenzoesäure-o-trimethylester I/2: 53 %

Beispiel 3

Elektrosynthese von 4-Methylbenzoesäure-o-trimethylester I/3 Zelle : ungeteilte Zelle mit 11 Elektroden

Anode : Graphit

Elektrolyt :

894 g (33,1 Gew.%) 4-Methylbenzaldehyddimethylacetal II/3

8,9 g (0,33 Gew.) Tris-(2,4-dibromphenyl)-amin

8,9 g (0,33 Gew.%) $KSO_3C_6H_5$

8,9 g (0,33 Gew.%) $NaOCH_3$

1.780,2 g (65,91 Gew.%) $CH_3OH$

Kathode : Graphit

Stromdichte : 2 $A/dm^2$

Elektrolyse mit 5,8 F/Mol 4-Methylbenzaldehyddimethylacetal II/3

Temperatur : 33°C

Durchfluß durch Zelle: 200 l/h

Aufarbeitung: analog Beispiel 1. Die fraktionierte Destillation wurde bei 2 mbar Kopfdruck und Kopftemperaturen von 70 bis 150°C durchgeführt. Hierbei erhielt man 230,3 g 4-Methylbenzaldehyddimethylacetal II/3, 240,1 g 4-Methylbenzoesäure-o-trimethylester I/3, 81,1 g 4-Methylbenzoesäuremethylester, 43,1 g 4-Methoximethylbenzaldehyddimethylacetal und 63,3 g Terephthalaldehyd-di-dimethylacetal.

Ergebnis:

Umsatz 4-Methylbenzaldehyddimethylacetal II/3: 74 %

Ausbeute 4-Methylbenzoesäure-o-trimethylester I/3: 23 %

Selektivität 4-Methylbenzoesäure-o-trimethylester I/3: 31 %

Beispiel 4

Elektrosynthese von 4-Methylbenzoesäure-o-trimethylester I/3 Zelle : ungeteilte Zelle mit 2 Elektroden

Anode : glasartiger Kohlenstoff

Elektrolyt :

1,66 g (1,66 Gew.%) 4-Methylbenzaldehyddimethylacetal II/3

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (52,1 Gew.%) $CH_3OH$

43,8 g (43,7 Gew.%) $CH_2Cl_2$

Kathode : Platin

Stromdichte : 0,5 bis 1,0 A/dm²

Elektrolyse mit 15,6 F/Mol 4-Methylbenzaldehyddimethylacetal II/3

Temperatur : 30°C

Aufarbeitung: Die Elektrolyselösung wurde auf die Hälfte des Volumens eingeengt, mit 20 ml $Na_2CO_3$-Lösung (5 %) versetzt und mit Pentan perforiert. Die Pentanlösung wurde über $MgSO_4$ getrocknet, das Lösungsmittel abgetrennt und der Rückstand destillativ gereinigt. Man erhielt 0,166 g 4-Methylbenzaldehyddimethylacetal II/3, 1,52 g 4-Methylbenzoesäure-o-trimethylester I/3, 0,16 g 1-(Dimethoxymethyl)-1,4-dimethoxy-4-methylcyclohexa-2,5-dien

Ergebnis:

Umsatz 4-Methylbenzaldehyddimethylacetal II/3: 90 %

Ausbeute 4-Methylbenzoesäure-o-trimethylester I/3: 78 %

Selektivität 4-Methylbenzoesäure-o-trimethylester I/3: 87 %

Beispiel 5

Elektrosynthese von Benzoesäure-o-trimethylester I/4 Zelle : ungeteilte Zelle mit 2 Elektroden

Anode : glasartiger Kohlenstoff

Elektrolyt :

1,53 g (1,53 Gew.%) Benzaldehyddimethylacetal II/4

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (52,2 Gew.%) $CH_3OH$

43,8 g (43,8 Gew.%) $CH_2Cl_2$

Kathode : Platin

Stromdichte : 0,5 bis 1,0 A/dm²

Elektrolyse mit 15,5 F/Mol Benzaldehyddimethylacetal II/4

Temperatur : 30°C

Aufarbeitung: Analog Beispiel 4. Man erhielt 0,055 g Benzaldehyddimethylacetal II/4, 0,161 g Benzoesäuremethylester, 1,508 g Benzoesäure-o-trimethylester.

Ergebnis:

Umsatz Benzaldehyddimethylacetal II/4: 96,4 %

Ausbeute Benzoesäure-o-trimethylester I/4: 82,5 %

Selektivität Benzoesäure-o-trimethylester I/4: 85,6 %

Beispiel 6

Elektrosynthese von 4-Chlorbenzoesäure-o-trimethylester I/5 Zelle : ungeteilte Zelle mit 2 Elektroden

Anode : glasartiger Kohlenstoff

Elektrolyt :

1,87 g (1,86 Gew.%) 4-Chlorbenzaldehyddimethylacetal II/5

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (52,0 Gew.%) $CH_3OH$

43,8 g (43,6 Gew.%) $CH_2Cl_2$

Kathode : Platin

Stromdichte : 0,5 bis 1,0 $A/dm^2$

Elektrolyse mit 25,9 F/Mol 4-Chlorbenzaldehyddimethylacetal II/5

Aufarbeitung: Analog Beispiel 4. Man erhielt 0,19 g 4-Chlorbenzaldehyddimethylacetal II/5, 0,197 g 4-Chlorbenzoesäuremethylester, 1,636 g 4-Chlorbenzoesäure-o-trimethylester I/5.

Ergebnis:

Umsatz 4-Chlorbenzaldehyddimethylacetal II/5: 89,7%

Ausbeute 4-Chlorbenzoesäure-o-trimethylester I/5: 75,5%

Selektivität 4-Chlorbenzoesäure-o-trimethylester I/5: 84 %

Beispiel 7

Elektrosynthese von 4-Brombenzoesäure-o-trimethylester I/6 Zelle : ungeteilte Zelle mit 2 Elektroden

Anode : glasartiger Kohlenstoff

Elektrolyt :

2,36 g (2,34 Gew.%) 4-Brombenzaldehyddimethylacetal II/6

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (51,8 Gew.%) $CH_3OH$

43,8 g (43,4 Gew.%) $CH_2Cl_2$

Kathode : Platin

Stromdichte : 0,5 bis 1,0 $A/dm^2$

Elektrolyse mit 18,2 F/Mol 4-Brombenzaldehyddimethylacetal II/6

Aufarbeitung: Analog Beispiel 4. Man erhielt 0,2 g 4-Brombenzaldehyddimethylacetal II/6, 0,078 g 4-Brombenzoesäuremethylester, 2,16 g 4-Brombenzoesäure-o-trimethylester I/6.

Ergebnis:

Umsatz 4-Brombenzaldehyddimethylacetal II/6: 92 %

Ausbeute 4-Brombenzoesäure-o-trimethylester I/6: 87,5 %

Selektivität 4-Brombenzoesäure-o-trimethylester I/6: 95 %

Beispiel 8

Elektrosynthese von 4-tert.Butylbenzoesäure-o-trimethylester I/7 Zelle : ungeteilte Zelle mit 2 Elektroden

Anode : glasartiger Kohlenstoff

Elektrolyt :

2,08 g (2,09 Gew.%) 4-tert.Butylbenzaldehyddimethylacetal II/7

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (51,9 Gew.%) $CH_3OH$

43,8 g (43,55 Gew.%) $CH_2Cl_2$

Kathode : Platin

Stromdichte : 0,5 bis 1,0 $A/dm^2$

Elektrolyse mit 12,9 F/Mol 4-tert.Butylbenzaldehyddimethylacetal II/7

Aufarbeitung: Analog Beispiel 4. Man erhielt 0,125 g 4-tert.Butylbenzoesäuremethylester, 2,16 g 4-tert.Butylbenzoesäure-o-trimethylester I/7.

Ergebnis:

Umsatz 4-tert.Butylbenzaldehyddimethylacetal II/7: 100 %

Ausbeute 4-tert.Butylbenzoesäure-o-trimethylester I/7: 90,4 %

Selektivität 4-tert.Butylbenzoesäure-o-trimethylester I/7: 90,4 %

Beispiel 9

Elektrosynthese von 4-Methoxibenzoesäure-o-trimethylester I/2 Zelle : ungeteilte Zelle mit 2 Elektroden

Anode : glasartiger Kohlenstoff

Elektrolyt :

1,83 g (1,82 Gew.%) 4-Methoxibenzaldehyddimethylacetal II/2

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (52,0 Gew.%) $CH_3OH$

43,8 g (43,7 Gew.%) $CH_2Cl_2$

Kathode : Platin

Stromdichte : 0,5 bis 1,0 $A/dm^2$

Elektrolyse mit 12,4 F/Mol 4-Methoxibenzaldehyddimethylacetal II/2

Aufarbeitung: Analog Beispiel 4. Man erhielt 0,22 g 4-Methoxybenzoesäuremethylester, 1,67 g 4-Methoxibenzoesäure-o-trimethylester I/2.

Ergebnis:

Umsatz 4-Methoxibenzaldehyddimethylacetal II/2: 100 %

Ausbeute 4-Methoxibenzoesäure-o-trimethylester I/2: 78,3 %

Selektivität 4-Methoxibenzoesäure-o-trimethylester I/3: 78,3 %

Beispiel 10

Elektrosynthese von 4-tert.Butyloxybenzoesäure-o-trimethylester I/1 Zelle : ungeteilte Zelle mit 2 Elektroden

Anode : glasartiger Kohlenstoff

Elektrolyt :

2,25 g (2,23 Gew.%) 4-tert.Butoxibenzaldehyddimethylacetal II/1

0,72 g (0,72 Gew.%) Tris-(2,4-dibromphenyl)-amin

1,6 g (1,6 Gew.%) $LiClO_4$

0,16 g (0,16 Gew.%) $NaOCH_3$

52,2 g (51,8 Gew.%) $CH_3OH$

43,8 g (43,5 Gew.%) $CH_2Cl_2$

Kathode : Platin

Stromdichte : 0,5 bis 1,0 $A/dm^2$

Elektrolyse mit 13,5 F/Mol 4-tert.Butoxibenzaldehyddimethylacetal II/1

Aufarbeitung: Analog Beispiel 4. Man erhielt 0,09 g 4-tert.Butoxibenzaldehyddimethylacetal II/1, 0,42 g 4-tert.Butoxibenzoesäuremethylester, 2,28 g 4-tert.Butoxibenzoesäure-o-trimethylester I/1.

Ergebnis:

Umsatz 4-tert.Butyloxibenzaldehyddimethylacetal II/1: 96 %

Ausbeute 4-tert.Butyloxibenzoesäure-o-trimethylester I/1: 89,4%

Selektivität 4-tert.Butyloxibenzoesäure-o-trimethylester I/1: 93 %

Beispiel 11

Elektrosynthese von 4-tert.Butylbenzoesäure-o-trimethylester I/9 Zelle : ungeteilte Zelle mit 2 Elektroden

Anode : glasartiger Kohlenstoff

Elektrolyt :

1,49 g (1,46 Gew.%) 4-tert.Butyltoluol II/9

0,72 g (0,70 Gew.%) Tris-(2,4-dibromphenyl)-amin

2,1 g (2,06 Gew.%) $Na_2CO_3$

52,2 g (51,1 Gew.%) $CH_3OH$

43,8 g (42,9 Gew.%) $CH_2Cl_2$

1.83 g (1,79 Gew.%) $NaClO_4$

Kathode : Platin

Stromdichte : 0,5 bis 1,0 $A/dm^2$

Elektrolyse mit 20 F/Mol 4-tert.Butyltoluol II/9

Aufarbeitung: Analog Beispiel 4. Man erhielt 0,2 g 4-tert.Butylbenzoesäuremethylester, 0,178 g 4-tert.Butylbenzylmethylether, 0,06 g 4-tert.Butylbenzaldehyddimethylacetal, 0,74 g 4-tert.Butylbenzoesäure-o-trimethylester I/3, 0,58 g 4-tert.Butyltoluol II/9.

Ergebnis:

Umsatz 4-tert.Butyltoluol II/9: 61 %

Ausbeute 4-tert.Butylbenzoesäure-o-trimethylester I/9: 31 %

Selektivität 4-tert.Butylbenzoesäure-o-trimethylester I/9: 51%

## Ansprüche

1. Verfahren zur Herstellung aromatischer Carbonsäureorthoester der allgemeinen Formel I

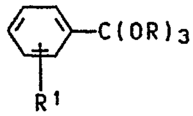

I,

in der R einen Alkylrest mit 1 bis 4 C-Atomen bedeutet und $R^1$ für ein Wasserstoffatom, Halogenatom oder einen Alkyl-, Aryl-, Heteroaryl-, Alkoxi-, Aryloxi-, Acyl-, Acyloxi-oder Cyanrest steht, dadurch gekennzeichnet, daß man Benzolderivate der allgemeinen Formel II

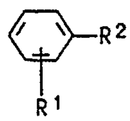

II,

in der $R^2$ für einen Methylrest oder einen Rest der Formel $-CH(OR)_2$ steht, in Gegenwart

a) eines Alkohols der Formel ROH, und

b) einer Triarylaminverbindung der allgemeinen Formel III

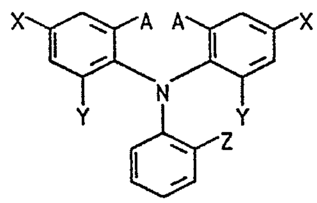

III,

in der beide A entweder Wasserstoffatome oder zusammen eine Einfachbindung, X ein Halogenatom oder einen $H_3COC$-, $NO_2$-oder NC-Rest, Y ein Wasserstoffatom, einen $-NO_2$-oder $CH_3COC$-Rest oder ein Halogenatom und Z ein Wasserstoffatom, einen $-NO_2$-Rest oder ein Halogenatom bedeuten, und

c) einer Base

elektrolysiert.

2. Verfahren nach Anspruch 1, dadurch gekennzeichnet, daß man als Alkohol Methanol verwendet.

3. Verfahren nach den Ansprüchen 1 und 2, dadurch gekennzeichnet, daß man als Triarylaminverbindung Tris-(2,4-dibromphenyl)-amin oder Tris-(2,4-dichlorphenyl)-amin verwendet.

4. Verfahren nach den Ansprüchen 1 bis 3, dadurch gekennzeichnet, daß man als Basen Alkoholate der allgemeinen Formel IV

$Me(OR)_x$ IV,

worin Me ein Alkali-oder Erdalkalimetall, x 1 oder 2 und R einen Alkylrest mit 1 bis 4 C-Atomen bedeuten, verwendet.

5. Verfahren nach den Ansprüchen 1 bis 4, dadurch gekennzeichnet, daß man einen Elektrolyten verwendet, der einen Gehalt an Benzaldehyddialkylacetal der Formel II von 1 bis 70 Gew.%, an Alkanol mit oder ohne Kolösungsmittel, von 30 bis 96 Gew.%, an Triarylaminverbindung von 0,5 bis 5,0 Gew.%, an Base von 0,05 bis 3,0 Gew.% und an Leitsalz von 0,5 bis 4,0 Gew.% aufweist.

6. Verfahren nach den Ansprüchen 1 bis 5, dadurch gekennzeichnet, daß man bei einer Stromdichte von 0,25 bis 10,0 A/dm² elektrolysiert.

7. 4-tert.Butoxibenzoesäure-o-trialkylester der allgemeinen Formel Ia

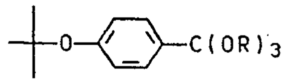

Ia,

in der R einen Alkylrest mit 1 bis 4 C-Atomen bedeutet.

8. 4-tert.Butoxibenzoesäure-o-trimethylester.

Europäisches Patentamt

# EUROPÄISCHER RECHERCHENBERICHT

Nummer der Anmeldung

EP 86 11 1019

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | INTERNATIONAL JOUNAL OF METHODS IN SYNTHETIC ORGANIC CHEMISTRY, Nr. 4, April 1978, Seiten 283,284; J.W. SCHEEREN et al.: "Electrochemical methoxylation of acetals. A simple procedure for the synthesis of orthoesters" --- | 1 | C 25 B 3/02<br>C 07 C 43/32 |
| P,Y | EP-A-0 179 289 (BASF)<br>* Insgesamt * --- | 1 | |
| A | EP-A-0 129 795 (BASF) ----- | | |
| | | | **RECHERCHIERTE SACHGEBIETE (Int. Cl.4)** |
| | | | C 25 B 3<br>C 07 C 43 |

Der vorliegende Recherchenbericht wurde für alle Patentansprüche erstellt.

| Recherchenort | Abschlußdatum der Recherche | Prüfer |
|---|---|---|
| DEN HAAG | 07-11-1986 | GROSEILLER PH.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82